# EUROPEAN PATENT SPECIFICATION

(11) **EP 4 299 705 B1**
(45) Date of publication and mention of the grant of the patent: **17.12.2025**
(21) Application number: 22181218.3
(22) Date of filing: 27.06.2022
(51) Int. Cl.: C11D 3/50, C11D 3/20, C11D 1/66

(54) **A PROCESS FOR MAKING A COMPOSITION COMPRISING ALKYL POLYGLYCOSIDE AND PERFUME**
VERFAHREN ZUR HERSTELLUNG EINER ZUSAMMENSETZUNG MIT ALKYLPOLYGLYCOSID UND PARFÜM
PROCÉDÉ DE FABRICATION D'UNE COMPOSITION COMPRENANT UN POLYGLYCOSIDE D'ALKYLE ET UN PARFUM

(43) Date of publication of application: 03.01.2024
(73) Proprietor: The Procter & Gamble Company, Cincinnati, OH 45202 (US)
(72) Inventor: BODET, Jean-Francois, 1853 Strombeek-Bever (BE); DELEERSNYDER, Geert André, 1853 Strombeek-Bever (BE); JEAN, Cindy, 1853 Strombeek-Bever (BE)
(74) Representative: P&G Patent Belgium UK

(56) References cited:
- JP-A- 2005 029 755
- US-A1- 2017 022 458

## Description

### FIELD OF THE INVENTION

The present invention relates to a process for making a composition, the composition comprising an alkyl polyglycoside surfactant and a perfume. It also relates to the use of a neutralizing agent to reduce perfume degradation in a composition comprising an alkyl polyglycoside surfactant and a perfume.

### BACKGROUND OF THE INVENTION

Users constantly look for simplification of the cleaning task. Some cleaning processes involve using a cleaning product and rinsing it. Cleaning habits are changing. In the past, time was dedicated to do deep cleaning, recently, people seem to prefer to clean little and often and try to maintain surfaces clean, in order to avoid the build-up of dirt, and to facilitate the subsequent cleaning tasks. Users nowadays also expect pleasant smell and shine of the surface cleaned. A dichotomy exists with aqueous cleaning compositions, the more perfume in the composition, the more emulsifier is required and this can negatively impact on shine. It can also be difficult to stabilize a relatively high level of perfume in an aqueous composition, especially in alkaline compositions. It is desirable to have a perfumed alkaline composition comprising an alkyl polyglycoside surfactant however, perfumes can easily degrade on alkaline compositions comprising an alkyl polyglycoside surfactant.

Alkyl glycosides are generally produced by reacting an alcohol with a reducing saccharide in the presence of an acid catalyst. The resulting solution is usually neutralized with an alkali, usually NaOH. Alkyl glycosides are soluble and stable in highly alkaline formulations. Usually, alkyl glycosides are supplied in the form of an aqueous alkaline solution ranging from 50 to 70% active. Alkyl glycosides solutions can negatively interact with perfumes components.

US 2017/022458 A1 discloses perfume compositions and delivery systems for use in a variety of consumer products, with the aim that a situs treated with such a product has superior cleanliness and is essentially aseptic. JP 2005/029755 A is directed to the provision of an aqueous, perfume-containing liquid composition, which does not cause deterioration of its container and avoids perfume leakage therefrom. The aqueous liquid composition comprises a hydrocarbon perfume, a specific nonionic surfactant, a water-soluble organic solvent and water.

A need remains for an alkaline stable composition comprising alkyl polyglycoside surfactant and relatively high level of perfume. There is also a need for a composition which provides improved scent during use and improved shine. There is also a need for a simplified method of cleaning a surface.

### SUMMARY OF THE INVENTION

According to the first aspect of the invention, there is provided a process for making a composition, the composition comprising:
a) an alkyl polyglycoside surfactant, preferably an alkyl polyglucoside; and
b) at least 0.1% by weight of the composition of a perfume wherein the perfume comprises at least 10% by weight of the perfume of perfume raw materials, the perfume raw materials comprising an ester functionality, an aldehyde functionality, a ketone functionality or a mixture thereof;
the composition has a pH equal or greater than about 7, preferably equal or greater than about 10 as measured at 20°C, the process comprises the steps of:
i) providing a concentrated aqueous solution comprising from 30 to 80% by weight of the solution of the alkyl polyglycoside;
ii) adding a neutralizing agent to the solution resulting from step i);
iii) adding the perfume to the mixture resulting from step ii).

The concentrated aqueous solution of step i) has a pH greater than about 11 as measured at 25°C.

It has surprisingly been found that the process of the invention gives rise to a composition in which the perfume is stable. The addition of the neutralizing agent contributes to the chemical stability of the perfume.

According to the second aspect of the invention, there is provided the use of a neutralizing agent in the process of the invention to reduce perfume degradation in the composition obtained according to the process.

The elements of the composition made according to the process of the invention described in relation to the first aspect of the invention apply *mutatis mutandis* to the other aspects of the invention.

### DETAILED DESCRIPTION OF THE INVENTION

Compositions made according to the process of the invention present good perfume stability, provide good cleaning, improved shine and very pleasant olfactory experience. The composition made according to the process of the invention is preferably clear and the perfume has good chemical stability. The composition preferably is isotropic and stable.

As defined herein, "essentially free of" a component means that no amount of that component is deliberately incorporated into the respective premix, or composition. Preferably, "essentially free of" a component means that no amount of that component is present in the respective premix, or composition.

As used herein, "isotropic" means a clear mixture, having little or no visible haziness, phase separation and/or dispersed particles, and having a uniform transparent appearance.

As defined herein, "physically stable" means that no visible phase separation is observed for a composition kept at 25°C for a period of at least two weeks, or at least four weeks, or greater than a month.

All percentages, ratios and proportions used herein are by weight percent of the composition, unless otherwise specified. All average values are calculated "by weight" of the composition, unless otherwise expressly indicated.

All measurements are performed at 25°C unless otherwise specified.

Unless otherwise noted, all component or composition levels are in reference to the active portion of that component or composition, and are exclusive of impurities, for example, residual solvents or by-products, which may be present in commercially available sources of such components or compositions.

### Process of the invention

The process of the invention comprises the steps of:
i) providing a concentrated aqueous solution, the concentrated aqueous solution comprises from 30 to 80%, preferably from 40% to 70% by weight of the solution of an alkyl polyglycoside surfactant, preferably an alkyl polyglucoside, more preferably a C8-10 alkyl polyglucoside;
ii) adding a neutralizing agent, preferably citric acid, to the solution of step i);
iii) adding the perfume to the mixture resulting from step ii); and wherein the concentrated aqueous solution of step i) has a pH greater than about 11 as measured at 25°C.

The concentrated aqueous solution comprising the alkyl polyglycoside surfactant usually has a starting pH of about 12 as measured at 25°C. The neutralizing agent, preferably citric acid, is added and mixed to allow neutralization to occur in order to bring the pH to 9 or less as measured at 25°C.

Preferably the pH of the starting concentrated aqueous solution is lowered, after adding the neutralizing agent, by about 1 or 2 or 3 or 4 or 5 pH units, more preferably the pH of the concentrated aqueous solution is from about 7 to about 8. Usually, the amount of neutralizing agent required is from 0.2 to 5%, more preferably from 0.4 to 2% by weight of the concentrated aqueous solution.

Preferably, the neutralizing agent also acts as a chelating agent for transition metals. Transition metals, such as iron, can be present in the initial concentrated aqueous solution as a byproduct of the synthesis of the alkyl polyglycoside surfactant. Chelation of transition metals may help the stability of the perfume.

The neutralizing agent may comprise an organic acid, an inorganic acid, or a mixture thereof. The neutralizing agent may be substantially free of trace transition metal impurities. A preferred organic acid of use herein has a pKa of less than 6. A suitable organic acid is selected from the group consisting of: citric acid, lactic acid, glycolic acid, succinic acid, glutaric acid and adipic acid and mixtures thereof. The preferred neutralizing agent for use herein is citric acid. A suitable inorganic acid can be selected from the group consisting of: hydrochloric acid, sulphuric acid, phosphoric acid and mixtures thereof. Citric acid is the preferred acid for use as neutralizing agent herein.

Suitable inorganic acids include phosphoric acid, sulfuric acid, urea-sulfuric acid, hydrochloric acid, sulfamic acid, methyl sulfuric acid, hypochlorous acid, sodium bisulfate, and the like. Suitable organic acids include polymeric acids comprising at least 3 carboxylic acid groups, C₁-C₁₁ organic acids comprising at least one carboxylic acid group, and organic acids that do not comprise carboxylic acid functional groups (such as imidazole derivatives or phenolic or polyphenolic compounds). Non-limiting examples of polymeric acids include polymers of acrylic acid, methacrylic acid, maleic acid, or itaconic acid or copolymers of acrylic acid, methacrylic acid, maleic acid, itaconic acid, or mixtures thereof. Polymeric acids may be homopolymers or copolymers having a molecular weight of about 500 g/mol or greater. The polymeric acid may have a molecular weight ranging from about 500 g/mol to about 1,000,000 g/mol, or from about 500 g/mol to about 100,000 g/mol, or from about 1,000 g/mol to about 20,000 g/mol. Copolymers may be random copolymers or block copolymers. In addition to monomer units comprising carboxylic acid groups, the copolymers may also include one or more other monomers, such as styrene, acrylic ester, acrylamide, olefin sulfonate, and olefin acetate.

Non-limiting examples of C₁-C₁₁ organic acids include formic acid, acetic acid, benzoic acid, malonic acid, citric acid, maleic acid, fumaric acid, succinic acid, lactic acid, malic acid, tartaric acid, gluconic acid, glutaric acid, adipic acid, 2-ethyl-1-hexanoic acid, heptanoic acid, octanoic acid, nonanoic acid, undecylenic acid, butane tetracarboxylic acid, and the like. The organic acid may be derived from a renewable, plant-based feedstock and produced using natural processes, such as fermentation; examples include bio-based acetic acid, bio-based citric acid, bio-based lactic acid and bio-based succinic acid, and the like. The organic acid may have food-use pedigree or be Generally Regarded As Safe (GRAS) or a food additive by the US Food & Drug Administration.

The neutralizing agent can be selected from the group consisting of formic acid, acetic acid, benzoic acid, malonic acid, citric acid, maleic acid, fumaric acid, hypochlorous acid, succinic acid, gluconic acid, glutaric acid, lactic acid, 2-ethyl-1-hexanoic acid, octanoic acid, nonanoic acid, peracetic acid, peroctanoic acid, undecylenic acid, and mixtures thereof, or the neutralizing agent can be selected from the group consisting of benzoic acid, citric acid, lactic acid succinic acid, maleic acid, succinic acid, octanoic acid, and mixtures thereof. Specially preferred for use herein is citric acid.

The process of the invention can comprise a step before step i) in which an alkaline solution is made, preferably the solution comprise an alkaline agent, more preferably monoethanol amine. The mixture resulting from step iii) is added to the alkaline solution, dyes and other cleaning adjuncts can then be added to the resulting mixture. The mixture is homogenized by mixing.

### Composition made by the process of the invention

The composition is preferably a treatment composition, more preferably a cleaning composition, more preferably a hard surface cleaning composition and most preferable an aqueous hard surface cleaning composition.

By "aqueous hard surface cleaning composition", it is meant herein a water based liquid composition for cleaning hard surfaces found in households, especially domestic households. Surfaces to be cleaned include kitchens and bathrooms, e.g., floors, walls, tiles, windows, cupboards, sinks, showers, shower plastified curtains, wash basins, WCs, fixtures and fittings and the like made of different materials like ceramic, vinyl, no-wax vinyl, linoleum, melamine, glass, steel, kitchen work surfaces, any plastics, plastified wood, metal or any painted or varnished or sealed surface and the like. Household hard surfaces also include household appliances including, but not limited to refrigerators, freezers, washing machines, automatic dryers, ovens, microwave ovens, dishwashers and so on. Such hard surfaces may be found both in private households as well as in commercial, institutional and industrial environments. The composition of the invention is especially suitable for the cleaning and treatment of kitchen surfaces.

The composition herein is an aqueous alkaline composition. By "aqueous composition" is herein meant a composition comprising more than 50%, preferably more than 60% by weight of the composition of water. The composition may comprise from 80% to 99.5%, preferably from 85% to 99% and more preferably from 94% to 98% by weight of the total composition of water.

The composition made by the process of the present invention has a pH which is greater or equal than 7, preferably greater or equal than 10, preferably greater than 10.5 as measured on the neat composition, at 25°C. It is believed that the greasy soil and particulate greasy soil cleaning performance is further improved at these preferred alkaline pH ranges, while surface safety is maintained. Accordingly, the compositions herein may further comprise a base to adjust pH as appropriate.

A suitable base to be used herein is an organic and/or inorganic base. Suitable bases of use herein are the caustic alkalis, such as sodium hydroxide, potassium hydroxide and/or lithium hydroxide, and/or the alkali metal oxides such, as sodium and/or potassium oxide or mixtures thereof. A preferred base is a caustic alkali, more preferably sodium hydroxide and/or potassium hydroxide.

Other suitable bases include ammonia, ammonium carbonate, potassium carbonate, sodium carbonate, sodium bicarbonate, and alkanolamines (such as monoethanolamine, triethanolamine, aminomethylpropanol, and mixtures thereof), nitrogenous buffers, and mixtures thereof. Suitable nitrogenous buffers include: ammonium or alkaline earth carbamates, guanidine derivatives, ammonium carbonate, ammonium bicarbonate, diammonium carbonate, ammonium hydroxide, ammonia (which forms ammonium hydroxide in situ when added to water) and mixtures thereof. Typical levels of such bases, when present, are from 0.01% to 5.0% by weight of the total composition, preferably from 0.05% to 3.0% and more preferably from 0.1% to 2.0 %.

All ratios are calculated as a weight/weight level, unless otherwise specified.

### Alkyl polyglycoside surfactant

The composition made by the process of the invention comprises an alkyl polyglycoside (APG). Preferred APGs include alkyl polyglucosides, which are characterized by the saccharide moiety being glucose. Preferred alkyl polyglucosides have naturally derived glucoside groups.

The alkyl polyglycosides, which can be used in the present invention, are fatty ether derivatives of saccharides or polysaccharides which are formed when a carbohydrate is reacted under acidic condition with a fatty alcohol through condensation polymerization. The APGs commonly are derived from corn-based carbohydrates and fatty alcohols from natural oils in animals, coconuts and palm kernels. Natural gas, or petroleum-based alcohols may also be used, particularly in shorter chain lengths. Such methods of deriving APGs are known in the art, for example, U.S. Pat. No. 5,003,057.

The alkyl polyglycoside that can be used in the present invention contains a hydrophilic group derived from carbohydrates and is composed of one or more anhydroglucose. Each of the glucose units can have two ether oxygens and three hydroxyl groups and a terminal hydroxyl group, imparting water solubility to the glycoside. The presence of the alkyl carbons leads to the hydrophobic activity. When carbohydrate molecules react with fatty alcohol molecules, alkyl poly glycoside molecules are formed with single or multiple anhydroglucose units, which are termed monoglycosides and polyglycosides, respectively. The final alkyl poly glycoside product typically has a distribution of varying concentration of glucose units (or degree of polymerization).

The APG used in the invention preferably comprises the saccharide or polysaccharide groups (i.e., mono-, di-, tri-, etc. saccharides) of hexose or pentose, and a fatty aliphatic group with 6 to 20 carbon atoms. Alkyl polyglycosides which can be used in the present invention are represented by the general formula of

(G)_{x-O-R}

where G is a moiety derived from a reducing saccharide containing 5 or 6 carbon atoms, e.g., pentose or hexose; R is fatty aliphatic group containing 6 to 20 carbon atoms; and x is the degree of polymerization (D.P.) of the polyglycoside, representing the number of monosaccharide repeating units in the polyglycoside. Generally, x is an integer on the basis of individual molecules, but because there are statistical variations in the manufacturing process of the APG, x may be a non-integer on an average basis when referred to APG used as an ingredient for composition of the present invention. In suitable APGs for use herein, x preferably has a value of less than about 5, and more preferably between about 0.5 and about 5. Even more preferably, x is less than about 2.5, and more preferably is within the range between about 1 and about 2.

Many commercially available alkyl polyglycosides may contain a blend of carbon lengths. Suitable alkyl polyglycosides include alkyl polyglycosides containing short chain carbons, such as chain lengths of less than C16. In one example, suitable alkyl polyglycosides include C8-C16 alkyl polyglycosides. Additional description of suitable alkyl polyglycosides are set forth, for example, in U. S. Patent Nos. 8,287,659 and 8,299,009, and U. S. Patent Application Serial Nos. 12/819,667, 12/884,638, 12/887,716, 13/597,380, 13/622,392, and 13/653,965.

Exemplary saccharides from which G is derived are glucose, fructose, mannose, galactose, talose, gulose, allose, altrose, idose, arabinose, xylose, lyxose and ribose. Because of the ready availability of glucose, glucose is preferred in the making of polyglycosides. The fatty aliphatic group, which is the substituent of the preferred polyglycoside, is preferably saturated, although unsaturated fatty group may be used.

In some embodiments, the APGs have an average degree of polymerization of saccharides from 1.4 to 1.7 and the chain lengths of the aliphatic groups are between C8 -16. Alkyl polyglycosides suitable for this invention can be described as illustrated in the following way: "C8-16 G 1.6" denotes a polyglycoside with an alkyl chain of 8 to 16 carbon atoms and an average degree of polymerization of 1.6 anhydroglucose units in the alkyl polyglucoside molecule. Commercially, alkyl polyglycosides can be provided as concentrated, aqueous solutions ranging from 50 to 70 wt. % active. Examples of suitable alkyl polyglucoside surfactants are the TRITON^{™} alkyl polyglucosides from Dow; Agnique PG, Disponil APG and Glucopon alkyl polyglucosides from BASF. Preferred alkyl polyglucoside surfactants are those where n is 8 to 12, more preferably 8 to 10. Examples of preferred polyglycosides include AG 6202, 2-ethylhexyl APG from Nouryon, Triton CG-50, C8-10 APG with low degree of glucose oligomerization from Dow, Triton GC-110, C8-10 APG with high degree of glucose oligomerization from Dow.

Preferably, the composition herein comprises from about 0.2 to about 5%, preferably from about 0.5 to about 2% by weight of the composition of APG, preferably from about 0.5 to about 2% by weight of the composition of alkyl polyglucoside. A C8-10 alkyl polyglucoside is specially preferred for use herein.

### Emulsifier

Emulsifiers for use herein include non-ionic surfactants, in particular alkoxylated nonionic surfactants. Alkoxylated nonionic surfactants include primary C₆-C₁₆ alcohol polyglycol ether i.e. ethoxylated alcohols having 6 to 16 carbon atoms in the alkyl moiety and 4 to 30 ethylene oxide (EO) units. When referred to for example C₉₋₁₄ it is meant average carbons and alternative reference to for example EO8 is meant average ethylene oxide units.

Suitable alkoxylated nonionic surfactants are according to the formula RO-(A)ₙH, wherein : R is a C₆ to C₁₈, preferably a C₈ to C₁₆, more preferably a C₈ to C₁₂ alkyl chain, or a C₆ to C₂₈ alkyl benzene chain; A is an ethoxy or propoxy or butoxy unit, and wherein n is from 1 to 30, preferably from 1 to 20 and, more preferably from 5 to 16 even more preferably from 7 to 12. Preferred R chains of use herein are the C₈ to C₂₂ alkyl chains. Even more preferred R chains of use herein are the C₁₂ to C₁₄ alkyl chains. R can be linear or branched alkyl chain.

Suitable ethoxylated nonionic surfactants of use herein are Dobanol^{®} 91-2.5 (HLB = 8.1; R is a mixture of C₉ and C₁₁ alkyl chains, n is 2.5), Dobanol^{®} 91-10 (HLB =14.2 ; R is a mixture of C₉ to C₁₁ alkyl chains, n is 10), Dobanol^{®} 91-12 (HLB =14.5 ; R is a mixture of C₉ to C₁₁ alkyl chains, n is 12), Greenbentine DE80 (HLB = 13.8, 98 wt% C10 linear alkyl chain, n is 8), Marlipal 10-8 (HLB = 13.8, R is a C10 linear alkyl chain, n is 8), Lialet^{®} 11-5 (R is a C₁₁ alkyl chain, n is 5), Isalchem^{®} 11-5 (R is a mixture of linear and branched C11 alkyl chain, n is 5), Lialet^{®} 11-21 (R is a mixture of linear and branched C₁₁ alkyl chain, n is 21), Isalchem^{®} 11-21 (R is a C₁₁ branched alkyl chain, n is 21), Empilan^{®} KBE21 (R is a mixture of C₁₂ and C ₁₄ alkyl chains, n is 21) or mixtures thereof. Preferred herein are Dobanol^{®} 91-5, Neodol^{®} 11-5, Lialet^{®} 11-21 Lialet^{®} 11-5 Isalchem^{®} 11-5 Isalchem^{®} 11-21 Dobanol^{®} 91-8, or Dobanol^{®} 91-10, or Dobanol^{®} 91-12, or mixtures thereof. These Dobanol^{®}/Neodol^{®} surfactants are commercially available from SHELL. These Lutensol^{®} surfactants are commercially available from BASF and these Tergitol^{®} surfactants are commercially available from Dow Chemicals.

Suitable chemical processes for preparing the alkoxylated nonionic surfactants of use herein include condensation of corresponding alcohols with alkylene oxide, in the desired proportions. Such processes are well known to the person skilled in the art and have been extensively described in the art, including the OXO process and various derivatives thereof. Suitable alkoxylated fatty alcohol nonionic surfactants, produced using the OXO process, have been marketed under the tradename NEODOL^{®} by the Shell Chemical Company. Alternatively, suitable alkoxylated nonionic surfactants can be prepared by other processes such as the Ziegler process, in addition to derivatives of the OXO or Ziegler processes.

Preferably, said alkoxylated nonionic surfactant is a C₉₋₁₁ EO5 alkylethoxylate, C₁₂₋₁₄ EO5 alkylethoxylate, a C₁₁ EO5 alkylethoxylate, C₁₂₋₁₄ EO21 alkylethoxylate, or a C₉₋₁₁ EO8 alkylethoxylate or a mixture thereof. Most preferably, said alkoxylated nonionic surfactant is a C₁₁ EO5 alkylethoxylate or a C₉₋₁₁ EO8 alkylethoxylate or a mixture thereof.

The composition can comprise from 0.05% to 2%, preferably from 0.08% to 0.5% by weight of the composition of alkoxylated nonionic surfactant, preferably from 0.08% to 0.5% by weight of the composition of ethoxylated alcohol.

### Additional Surfactant:

The hard surface cleaning composition may comprise up to 1% by weight of an additional surfactant, preferably selected from: anionic, amphoteric, zwitterionic, and mixtures thereof. The hard surface cleaning composition can comprise from 0.01% to 1% by weight of the additional surfactant. Preferably, the composition is substantially free of surfactants other than alkyl polyglycosides and alkoxylated non-ionic surfactants.

### Perfume

The composition comprises a perfume. The perfume is a mixture of odorant raw materials, such as aromatic natural oils and aromatic chemicals, which taken together form a complex scent that delivers a number of benefits. These benefits may include the coverage of product base odor, scenting the product itself, and lingering scent radiating from the surface into the air after cleaning. When the composition is sprayed, the benefit may also include the delivery of scent to the air when spraying the composition on a surface, and the delivery of scent to the air while wiping the composition on the surface. The perfume may comprise at least 3, at least 5, at least 7, at least 11, or at least 15 perfume raw materials.

As used herein, the term "perfume raw material" (or "PRM") refers to compounds having a molecular weight of at least about 100 g/mol and which are useful in imparting an odor, fragrance, essence, or scent, either alone or with other perfume raw materials. Typical PRMs comprise inter alia alcohols, ketones, aldehydes, esters, ethers, nitrites and alkenes, such as terpene. A listing of common PRMs can be found in various reference sources, for example, "Perfume and Flavor Chemicals", Vols. I and II; Steffen Arctander Allured Pub. Co. (1994) and "Perfumes: Art, Science and Technology", Miller, P. M. and Lamparsky, D., Blackie Academic and Professional (1994). At least some of the PRMs of the present perfume include esters, aldehydes and ketones, but such PRMs are typically used in combination with other PRMs, which may include other functional groups, such as alcohols.

The PRMs may be characterized by their boiling points (B.P.) measured at the normal pressure (760 mmHg), and their octanol/water partitioning coefficient (P), which may be described in terms of logP, determined according to the test method described in Test methods section. Based on these characteristics, the PRMs may be categorized as Quadrant I, Quadrant II, Quadrant III, or Quadrant IV perfumes, as described in more detail below. A perfume having a variety of PRMs from different quadrants may be desirable, for example, to provide fragrance benefits at different touchpoints during normal usage of the composition.

Perfume raw materials having a boiling point B.P. lower than about 250°C and a logP lower than about 3 are known as Quadrant I perfume raw materials. Quadrant 1 perfume raw materials are preferably limited to less than 30% of the perfume composition. Perfume raw materials having a B.P. of greater than about 250°C and a logP of greater than about 3 are known as Quadrant IV perfume raw materials, perfume raw materials having a B.P. of greater than about 250°C and a logP lower than about 3 are known as Quadrant II perfume raw materials, perfume raw materials having a B.P. lower than about 250°C and a logP greater than about 3 are known as a Quadrant III perfume raw materials. Suitable Quadrant I, II, III, and IV perfume raw materials are disclosed in U.S. Patent 6,869,923 B1.

The perfume comprised in the composition made by the process of the invention comprises at least 10% by weight of the perfume of perfume raw materials comprising an ester functionality, an aldehyde functionality, a ketone functionality or a mixture thereof. Preferably, the perfume comprises at least 10% by weight of the perfume of perfume raw materials comprising an ester functionality. Preferably, the perfume comprises at least 5% by weight of the perfume of perfume raw materials comprising an aldehyde functionality and preferably at least 5% by weight of the perfume of perfume raw materials comprising a ketone functionality. More preferably, the perfume comprises at least 25% by weight of the perfume of a mixture of perfume raw materials comprising ester, aldehyde and ketone functionalities.

### Perfume raw materials comprising an ester functionality

Examples of perfume raw materials comprising an ester functionality include acetates, anthranilates, benzoates, butyrates, cinnamates, formates, isobutyrates, phenylacetates and propionates. Some ester-containing perfume raw materials include: anisyl acetate, benzyl benzoate, cedryl formate, cinnamyl isobutyrate, citronellyl acetate, citronellyl formate, dihydrocarvyl acetate, diheptyl acetate, ethyl phenyl acetate, furfuryl phenyl acetate, geranyl benzoate, geranyl butyrate, hexyl caproate, isobutyl phenol acetate, isononyl acetate, isononyl propionate, linalyl acetate, linalyl formate, phenoxy ethyl isobutyrate, benzyl acetate, tricyclodecenyl acetate, tricyclodecenyl propionate, geranyl acetate, phenyl ethyl acetate, pseudo linalyl acetate and methyl benzoate and mixtures thereof.

### Perfume raw materials comprising an aldehyde functionality

Perfume raw materials comprising an aldehyde functionality may comprise from about eight to about twelve carbon atoms; such PRMs may be called C8-C12 aldehydes.

As examples, the structures of two aldehydes, each having twelve carbon atoms (e.g., both are C12 aldehydes), are provided below: dodecanal (a linear aldehyde-containing PRM; also known as dodecyl aldehyde or lauryl aldehyde) and 2-methylundecanal (a non-linear aldehyde-containing PRM; also known as methyl nonyl acetaldehyde, or "MNA") are shown below.

| **PRM Name** | **Structure** |
|---|---|
| dodecanal (linear) | 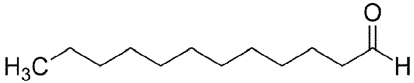 |
| 2-methylundecanal (or "MNA") (non-linear) | 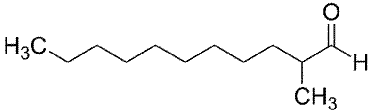 |

The perfume may comprise an aldehyde-containing perfume raw material (or residue thereof) selected from the group consisting of: decyl aldehyde, intreleven aldehyde, lauric aldehyde, methyl nonyl acetaldehyde, nonyl aldehyde, octyl aldehyde, undecyl aldehyde, undecylenic aldehyde, trans-2 hexenal, aqual, pinyl isobutyraldehyde alpha, calypsone, nironal, noreenal, nympeal, cyclamen aldehyde, ligustral, neo hivemal, p.t. bucinal (lilial), trans-2-docecenal, tangerinal, anisic aldehyde, iso hexenyl cyclohexenyl carboxaldehyde, melonal, citral, dupical, vanillin, ethyl vanillin, helional, heliotropin, hexyl cinnamic aldehyde, hydroxycitronellal, citronellal, floral ozone, starfleur, 4,7-Methano-1H-indene-2-carboxaldehyde, octahydro-5-methoxy- (Scentenal^{®} 981810), adoxal, mefranal, florhydral, bergamal, canthoxal, cinnamic aldehyde, citrathal, citronellyl oxyacetaldehyde, cyclemax, floral super, 3-Cyclohexene-1-propanal,beta,4-dimethyl- (Liminal^{®} 955374), lyral, phenyl acetaldehyde, pino acetaldehyde, trans-4-decenal, vemaldehyde, Octahydro-1H-4,7-methanoindene-5-carbaldehyde (Vertral^{®}), and mixtures thereof.

### Perfume raw materials comprising a ketone functionality

Perfume raw materials comprising a ketone functionality include, for example, methyl beta-naphthyl ketone, musk indanone (1,2,3,5,6,7-Hexahydro-1,1,2,3,3-pentamethyl-4H-inden-4-one), tonalide (6-Acetyl-1,1,2,4,4,7-hexamethyltetralin), alpha-damascone, beta-damascone, deltadamascone, iso-damascone, damascenone, methyl dihydrojasmonate, menthone, carvone, camphor, kovone (3,4,5,6,6-pentamethylhept-3-en-2-one), Fenchone, alpha-ionone, beta-ionone, gamma-methyl ionone, fleuramone (2-heptylcyclopene-tanone), dihydrojasmone, cis-jasmone, iso-E-Super (1- (1,2,3,4,5,6 J, 8-octahydro-2,3.8,8-tetramethyl-2-naphthalenyl) -ethan-1-one (and isomers), methyl cedrenyl ketone, acetophenone, methyl acetophenone, para-methoxy acetophenone, methyl beta-naphthyl ketone, benzyl acetone, benzophenone, para-hydroxyphenyl butanone, Selenium ketone (3-methyl-5-propyl-2-cyclohexenone), 6-isopropyldecahydro-2-naphthone, dimethyloctenone, Fresnel menthe (2-butan-2-yl-cyclohexan-1-one), 4- (1-ethoxyvinyl) -3,3,5,5-tetramethylcyclohexanone, methylheptenone, 2- (2- (4-methyl-3-cyclohexen-1-yl) propyl) cyclopentanone, 1- (p-Menthen-6 (2) yl) -1-propanone, 4- (4-hydroxy-3-methoxyphenyl) -2-butanone, 2-Acetyl-3,3-dimethylnorbornane, 6,7-Dihydro-1,1,2,3,3-pentamethyl-4 (5 H) -indanone, 4-damascol, Dulcinyl (4- (1,3-benzodioxol-5-yl) butan-2-one), hexalone (1- (2,6,6-trimethyl-2-cyclohexen-1-yl) -1,6-heptadien-3-one), IsocyclemonE (2-acetonaphthone-1,2,3,4,5,6,7,8-octahydro-2,3,8,8-tetramethyl), methyl nonyl ketone, methyl cyclocitrone, methyl lavendel ketone, orivone (4-tert-amylcyclohexanone), 4-tert-butylcyclohexanone, Delphon (2-pentylcyclopentanone), muscone (CAS 541-91-3), neobutenone (1-(5,5-dimethyl-1- cyclohexenyl)pent-4-en-1-one), plicatone (CAS 41724-19-0), veloutone (2,2,5-trimethyl-5- pentylcyclopentan-1-one). 2,4,4,7-tetramethyloct-6-en-3-one and tetrameran (6,10- dimethylundecen-2-one).

Perfume raw materials comprising a ketone functionality include alpha-damascone, beta-damascone, gamma-damascone, deltadamascone, alpha-ionone, beta-ionone, gamma-ionone, delta-ionone, beta-damascenone, 3-methyl-5-propyl-2-cyclohexen-1-one, 1 (6),8-P-menthadien-2-one, 2,5-dimethyl-5- phenyl-1-hexen-3-one, 1-(5,5-dimethyl-1-cyclohexen-1-yl)-4-penten-1-one, 8 or 10-methyl-alpha-ionone, 2-octenal, 1-(2,2,3,6-tetramethyl-1-cyclohexyl)-2-buten-1-one, 4-(2,2,3,6-tetramethyl-1-cyclohexyl)-3-buten-2-one, 2-cyclopentadecen-1-one, nootkatone, cinnamic aldehyde, 2,6,6-trimethyl-bicyclo[3. 1 .1 ]heptane-3-spiro-2'cyclohexen-4'-one, ethyl 2,4-deca-dienoate, ethyl 2-octenoate, methyl 2-nonenoate, ethyl 2,4-undecadienoate and methyl 5,9-dimethyl-2,4,8- decatrienoate.

The composition made by the process of the invention comprises at least 1% by weight of perfume and may comprise to 1%, or from 0.1% to 0.8% by weight of perfume.

### Optional ingredients:

The liquid hard surface cleaning compositions may comprise a variety of optional ingredients depending on the technical benefit aimed for and the surface treated. Suitable optional ingredients of use herein include builders, polymers, buffers, bactericides, hydrotropes, colorants, stabilisers, radical scavengers, abrasives, soil suspenders, brighteners, anti-dusting agents, dispersants, dye transfer inhibitors, pigments, silicones and/or dyes.

Preferred compositions comprise:
a) from about 0.2% to about 2% by weight of the composition of alkyl polyglycoside surfactant, preferably an alkyl polyglucoside, more preferably a C8-10 alkyl polyglucoside;
b) from about 0.05% to about 0.3% by weight of the composition of ethoxylated alcohol, preferable C12-C14 ethoxylated alcohol with a degree of ethoxylation of from 5 to 15, preferably from 8 to 12;
c) from 0.1 to 2% by weight of the composition of perfume.

The composition can be in the form of a ready-to-use composition or in the form of a concentrated composition.

A preferred composition for use herein, especially as a sprayable composition, comprises:
a) from about 0.5 to 2% by weight of the composition of the alkyl polyglycoside, preferably an alkyl polyglucoside, more preferably a C8-10 alkyl polyglucoside;
b) from about 0.1 to 0.5% by weight of the composition of an emulsifier, preferably a non-ionic surfactant, more preferably an alcohol ethoxylated; and
c) from about 0.1 to 0.5% by weight of the composition of the perfume.

A preferred composition for use herein, especially as a concentrated composition to be diluted in water before use, comprises:
a) from about 5 to 20% by weight of the composition of the alkyl polyglycoside, preferably an alkyl polyglucoside, more preferably a C8-10 alkyl polyglucoside;
b) from about 1 to 5% by weight of the composition of an emulsifier, preferably a non-ionic surfactant, more preferably an alcohol ethoxylated; and
c) from about 1 to 5% by weight of the composition of the perfume.

### Method of cleaning a surface:

A method of cleaning (not according to the invention) comprises the steps of:
a) contacting the surface with the composition made by the process of the present invention;
b) wiping the surface.

Preferably, the method does not require rinsing.

The composition can be a "ready-to-use" composition, where dilution is not necessary. Such ready-to-use compositions can be comprised in a spray container.

Said method of cleaning a hard surface may include the steps of applying, preferably spraying, said liquid composition onto said hard surface, leaving said liquid composition to act onto said surface for a period of time to allow said composition to act, with or without applying mechanical action.

### Methods:

### pH measurement:

The pH is measured on the neat composition, at 25°C, using a Sartarius PT-10P pH meter with gel-filled probe (such as the Toledo probe, part number 52 000 100), calibrated according to the instructions manual.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### EXAMPLE

Three compositions comprising C8-C10 alkylpolyglucoside (APG) were made by simple mixing (Compositions A* to C). Composition A* was made using a C8-C10 alkylpolyglucoside solution having pH 12. Composition B* was made using a C8-C10 alkylpolyglucoside solution having pH 7. Composition C was made by neutralizing a C8-C10 alkylpolyglucoside solution having an initial pH 12 with citric acid to bring the pH down to pH 7.

| | A* | B* | C |
|---|---|---|---|
| | wt% | wt% | wt% |
| Monoethanolamine | 0.3 | 0.3 | 0.3 |
| Sodium carbonate | 0.1 | 0.1 | 0.1 |
| Propylene glycol n-butyl ether | 0.2 | 0.2 | 0.2 |
| C8-C10 APG (pH 12) | 0.12 | | |
| C8-C10 APG (pH 7) | | 0.15 | |
| C8-C10 APG (pre-neutralized with citric acid to pH 7) | - | - | 1.0 |
| C12-14 EO 11¹ | 0.2 | 0.2 | 0.2 |
| Perfume (as show in table below) | 0.2 | 0.2 | 0.2 |
| Final composition pH | 11 | 11 | 11 |

| | | | |
|---|---|---|---|
| * Comparative ¹ nonionic surfactant commercially available from Sasol | | | |

The composition of the perfume is shown in the table below.

| | wt% |
|---|---|
| Terpenes | 34.4 |
| Ester | 19.9 |
| Aldehydes | 7.2 |
| Ether/ketone | 14.9 |
| Alcohol | balance |

The compositions were stored for 2 weeks at 25°C in PET bottles. The perfume was extracted via automatic hexane extraction. The components of the perfume were analysed via gas chromatography mass spectrometry using an Agilent 7890B GC + 5977B MSD.

The table below shows the loss of perfume of compositions A* to C after two weeks.

| Loss of perfume (wt%) | A* | B* | C |
|---|---|---|---|
| Terpenes | 52.2 | 34 | 34 |
| Ester | 84.7 | 65.3 | 24.7 |
| Aldehydes | 74.9 | 56.8 | 59.8 |
| Ether/ketone | 63.8 | 16 | 7 |
| Alcohol | balance | balance | balance |

The compositions made with APG at neutral pH shows better stability of esters, ether and ketones versus a composition made from an alkaline APG. A composition made by pre-neutralization of alkaline APG with citric acid shows superior stability of esters, ethers and ketone versus both a composition made with APG neutral pH per design and composition made from alkaline APG.

## Claims

1. A process for making a composition, the composition comprising:
a) an alkyl polyglycoside surfactant; and
b) at least 0.1% by weight of the composition of a perfume wherein the perfume comprises at least 10% by weight thereof of perfume raw materials selected from the group consisting of perfume raw materials comprising an ester functionality, an aldehyde functionality, a ketone functionality and a mixture thereof;
wherein the composition has a pH equal or greater than about 7, preferably equal or greater than about 10 as measured at 25°C, the process comprising the steps of:
i) providing a concentrated aqueous solution comprising from 30 to 80% by weight of the alkyl polyglycoside;
ii) adding a neutralizing agent to the solution of step i);
iii) adding the perfume to the mixture resulting from step ii);
and wherein the concentrated aqueous solution of step i) has a pH greater than about 11 as measured at 25°C.

2. A process according to the preceding claim wherein the addition of the neutralizing agent to the concentrated aqueous solution reduces the pH of the solution by at least 3 pH units, preferably by at least 4 pH units.

3. A process according to any of the preceding claims wherein the pH of the concentrated aqueous solution after the neutralization of step ii) is from about 6.5 to about 8.5.

4. A process according to any of the preceding claims wherein the neutralizing agent comprises citric acid.

5. A process according to any of the preceding claims wherein the perfume comprises at least 10% by weight thereof of perfume raw materials comprising an ester functionality.

6. A process according to any of the preceding claims wherein the perfume comprises at least 5% by weight thereof of perfume raw materials comprising an aldehyde functionality and preferably at least 5% of perfume raw materials comprising a ketone functionality.

7. A process according to any of the preceding claims wherein the perfume comprises at least 25% by weight thereof of a mixture of perfume raw materials comprising ester, aldehyde and ketone functionalities.

8. A process according to any of the preceding claims wherein the perfume is added to the mixture resulting from step ii) in the form of a pre-mix comprising an emulsifier, wherein the emulsifier preferably comprises a non-ionic surfactant.

9. A process according to any of the preceding the claims comprising the step of making an alkaline aqueous solution, adding the mixture resulting from step iii) to the alkaline aqueous solution and optionally adding dyes to the resulting mixture.

10. A process according to any of the preceding claims wherein the composition comprises an emulsifier and wherein the alkyl polyglycoside surfactant and the emulsifier are present in a weight ratio of at least 4:1.

11. A process according to the preceding claim wherein the emulsifier comprises a non-ionic surfactant, preferably an alkoxylated alcohol, preferably the alkoxylated alcohol comprises from 6 to 16 carbon atoms and from 2 to 12 alkoxy groups.

12. A process according to any of the preceding claims wherein the composition comprises:
a) from about 0.5 to 2% by weight of the composition of the alkyl polyglycoside;
b) from about 0.1 to 0.5% by weight of the composition of an emulsifier, preferably a non-ionic surfactant, more preferably an alcohol ethoxylated; and
c) from about 0.1 to 0.5% by weight of the composition of the perfume.

13. A process according to any of claims 1 to 11 wherein the composition comprises:
a) from about 5 to 20% by weight of the composition of the alkyl polyglycoside;
b) from about 1 to 5% by weight of the composition of an emulsifier, preferably a non-ionic surfactant, more preferably an alcohol ethoxylated; and
c) from about 1 to 5% by weight of the composition of the perfume.

14. Use of a neutralizing agent in a process according to any of claims 1 to 13 to reduce perfume degradation in the composition obtained according to the process.

## Patentansprüche

1. Verfahren zum Herstellen einer Zusammensetzung, die Zusammensetzung umfassend:
a) ein Alkylpolyglycosidtensid; und
b) zu wenigstens 0,1 Gew.-% der Zusammensetzung einen Duftstoff, wobei der Duftstoff zu wenigstens 10 Gew.-% davon Duftstoffrohmaterialien umfasst, ausgewählt aus der Gruppe, bestehend aus Duftstoffrohmaterialien, umfassend eine Esterfunktionalität, eine Aldehydfunktionalität, eine Ketonfunktionalität und eine Mischung davon;
wobei die Zusammensetzung einen pH-Wert von gleich oder größer als etwa 7, vorzugsweise gleich oder größer als etwa 10, gemessen bei 25 °C, aufweist, das Verfahren umfassend die Schritte:
i) Bereitstellen einer konzentrierten wässrigen Lösung, umfassend von zu 30 bis 80 Gew.-% das Alkylpolyglycosid;
ii) Zugeben eines Neutralisationsmittels zu der Lösung von Schritt i);
iii) Zugeben des Duftstoffs zu der aus Schritt ii) resultierenden Mischung;
und wobei die konzentrierte wässrige Lösung von Schritt i) einen pH-Wert von größer als etwa 11, gemessen bei 25 °C, aufweist.

2. Verfahren nach dem vorstehenden Anspruch, wobei die Zugabe des Neutralisationsmittels zu der konzentrierten wässrigen Lösung den pH-Wert der Lösung um wenigstens 3 pH-Einheiten, vorzugsweise um wenigstens 4 pH-Einheiten, verringert.

3. Verfahren nach einem der vorstehenden Ansprüche, wobei der pH-Wert der konzentrierten wässrigen Lösung nach der Neutralisation von Schritt ii) von etwa 6,5 bis etwa 8,5 beträgt.

4. Verfahren nach einem der vorstehenden Ansprüche, wobei das Neutralisationsmittel eine Citronensäure umfasst.

5. Verfahren nach einem der vorstehenden Ansprüche, wobei der Duftstoff zu wenigstens 10 Gew.-% davon Duftstoffrohmaterialien, umfassend eine Esterfunktionalität, umfasst.

6. Verfahren nach einem der vorstehenden Ansprüche, wobei der Duftstoff zu wenigstens 5 Gew.-% davon Duftstoffrohmaterialen, umfassend eine Aldehydfunktionalität, und vorzugsweise zu wenigstens 5 Gew.-% Duftstoffrohmaterialen, umfassend eine Ketonfunktionalität, umfasst.

7. Verfahren nach einem der vorstehenden Ansprüche, wobei der Duftstoff zu wenigstens 25 Gew.-% davon eine Mischung von Duftstoffrohmaterialien, umfassend Ester-, Aldehyd- und Ketonfunktionalität, umfasst.

8. Verfahren nach einem der vorstehenden Ansprüche, wobei der Duftstoff zu der aus Schritt ii) resultierenden Mischung in der Form eines Vorgemischs, umfassend einen Emulgator, zugegeben wird, wobei der Emulgator vorzugsweise ein nichtionisches Tensid umfasst.

9. Verfahren nach einem der vorstehenden Ansprüche, umfassend die Schritte des Herstellens einer alkalischen wässrigen Lösung, des Zugebens der aus Schritt iii) resultierenden Mischung zu der alkalischen wässrigen Lösung und wahlweise des Zugebens von Farbstoffen zu der resultierenden Mischung.

10. Verfahren nach einem der vorstehenden Ansprüche, wobei die Zusammensetzung einen Emulgator umfasst und wobei das Alkylpolyglycosidtensid und der Emulgator in einem Gewichtsverhältnis von wenigstens 4 : 1 gegenwärtig sind.

11. Verfahren nach dem vorstehenden Anspruch, wobei der Emulgator ein nichtionisches Tensid, vorzugsweise einen alkoxylierten Alkohol, umfasst, wobei der alkoxylierte Alkohol vorzugsweise von 6 bis 16 Kohlenstoffatome und von 2 bis 12 Alkoxygruppen umfasst.

12. Verfahren nach einem der vorstehenden Ansprüche, wobei die Zusammensetzung umfasst:
a) von zu etwa 0,5 bis 2 Gew.-% der Zusammensetzung das Alkylpolyglycosid;
b) von zu etwa 0,1 bis 0,5 Gew.-% der Zusammensetzung einen Emulgator, vorzugsweise ein nichtionisches Tensid, mehr bevorzugt einen ethoxylierten Alkohol; und
c) von zu etwa 0,1 bis 0,5 Gew.-% der Zusammensetzung den Duftstoff.

13. Verfahren nach einem der Ansprüche 1 bis 11, wobei die Zusammensetzung umfasst:
a) von zu etwa 5 bis 20 Gew.-% der Zusammensetzung das Alkylpolyglycosid;
b) von zu etwa 1 bis 5 Gew.-% der Zusammensetzung einen Emulgator, vorzugsweise ein nichtionisches Tensid, mehr bevorzugt einen ethoxylierten Alkohol; und
c) von zu etwa 1 bis 5 Gew.-% der Zusammensetzung den Duftstoff.

14. Verwendung eines Neutralisationsmittels in einem Verfahren nach einem der Ansprüche 1 bis 13, um eine Duftstoffdegradation in der gemäß dem Verfahren erhaltenen Zusammensetzung zu verringern.

## Revendications

1. Procédé permettant de fabriquer une composition, la composition comprenant :
a) un agent tensioactif alkyl polyglycoside ; et
b) au moins 0,1 % en poids de la composition d'un parfum dans lequel le parfum comprend au moins 10 %, en poids de celui-ci, de matières premières de parfum choisies dans le groupe constitué de matières premières de parfum comprenant une fonctionnalité ester, une fonctionnalité aldéhyde, une fonctionnalité cétone et un mélange de celles-ci ;
dans lequel la composition a un pH égal ou supérieur à environ 7, de préférence égal ou supérieur à environ 10 tel que mesuré à 25 °C, le procédé comprenant les étapes consistant à :
i) fournir une solution aqueuse concentrée comprenant de 30 à 80 % en poids de l'alkyl polyglycoside ;
ii) ajouter un agent neutralisant à la solution de l'étape i) ;
iii) ajouter le parfum au mélange résultant de l'étape ii) ;
et dans lequel la solution aqueuse concentrée de l'étape i) a un pH supérieur à environ 11 tel que mesuré à 25 °C.

2. Procédé selon la revendication précédente dans lequel l'ajout de l'agent neutralisant à la solution aqueuse concentrée réduit le pH de la solution d'au moins 3 unités de pH, de préférence d'au moins 4 unités de pH.

3. Procédé selon l'une quelconque des revendications précédentes, dans lequel le pH de la solution aqueuse concentrée après la neutralisation de l'étape ii) va d'environ 6,5 à environ 8,5.

4. Procédé selon l'une quelconque des revendications précédentes, dans lequel l'agent neutralisant comprend de l'acide citrique.

5. Procédé selon l'une quelconque des revendications précédentes, dans lequel le parfum comprend au moins 10 %, en poids de celui-ci, de matières premières de parfum comprenant une fonctionnalité ester.

6. Procédé selon l'une quelconque des revendications précédentes, dans lequel le parfum comprend au moins 5 %, en poids de celui-ci, de matières premières de parfum comprenant une fonctionnalité aldéhyde et de préférence au moins 5 % de matières premières de parfum comprenant une fonctionnalité cétone.

7. Procédé selon l'une quelconque des revendications précédentes, dans lequel le parfum comprend au moins 25 %, en poids de celui-ci, d'un mélange de matières premières de parfum comprenant des fonctionnalités ester, aldéhyde et cétone.

8. Procédé selon l'une quelconque des revendications précédentes, dans lequel le parfum est ajouté au mélange résultant de l'étape ii) sous la forme d'un prémélange comprenant un émulsifiant, dans lequel l'émulsifiant comprend de préférence un agent tensioactif non ionique.

9. Procédé selon l'une quelconque des revendications précédentes comprenant l'étape consistant à fabriquer une solution aqueuse alcaline, à ajouter le mélange résultant de l'étape iii) à la solution aqueuse alcaline et à ajouter facultativement des teintures au mélange résultant.

10. Procédé selon l'une quelconque des revendications précédentes, dans lequel la composition comprend un émulsifiant et dans lequel l'agent tensioactif alkyl polyglycoside et l'émulsifiant sont présents dans un rapport pondéral d'au moins 4:1.

11. Procédé selon la revendication précédente dans lequel l'émulsifiant comprend un agent tensioactif non ionique, de préférence un alcool alcoxylé, de préférence l'alcool alcoxylé comprend de 6 à 16 atomes de carbone et de 2 à 12 groupes alcoxy.

12. Procédé selon l'une quelconque des revendications précédentes, dans lequel la composition comprend :
a) d'environ 0,5 à 2 % en poids de la composition de l'alkyl polyglycoside ;
b) d'environ 0,1 à 0,5 % en poids de la composition d'un émulsifiant, de préférence un agent tensioactif non ionique, plus préférablement un alcool éthoxylé ; et
c) d'environ 0,1 à 0,5 % en poids de la composition du parfum.

13. Procédé selon l'une quelconque des revendications 1 à 11 dans lequel la composition comprend :
a) d'environ 5 à 20 % en poids de la composition de l'alkyl polyglycoside ;
b) d'environ 1 à 5 % en poids de la composition d'un émulsifiant, de préférence un agent tensioactif non ionique, plus préférablement un alcool éthoxylé ; et
c) d'environ 1 à 5 % en poids de la composition du parfum.

14. Utilisation d'un agent neutralisant dans un procédé selon l'une quelconque des revendications 1 à 13 pour réduire une dégradation de parfum dans la composition obtenue selon le procédé.
